(19) Europäisches Patentamt
European Patent Office
Office européen des brevets

(11) **EP 2 508 197 A1**

(12) **EUROPEAN PATENT APPLICATION**

(43) Date of publication:
**10.10.2012 Bulletin 2012/41**

(51) Int Cl.:
**A61K 38/21** $^{(2006.01)}$     **A61K 39/00** $^{(2006.01)}$

(21) Application number: **11305408.4**

(22) Date of filing: **07.04.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **NEOVACS**
**75014 Paris (FR)**

(72) Inventors:
• **Grouard-Vogel, Géraldine**
**75014 Paris (FR)**

• **Dhellin, Olivier**
**75020 Paris (FR)**
• **Fanget, Bernard**
**42800 Châteauneuf (FR)**
• **Vandepapeliére, Pierre**
**5021 Bonnine (BE)**

(74) Representative: **Icosa**
**83 avenue Denfert-Rochereau**
**75014 Paris (FR)**

(54) **Method for treating IFNalpha related conditions**

(57)    The present invention relates to an immunogenic product comprising IFN$\alpha$ coupled to a carrier protein molecule that is capable to induce in vivo anti-IFN$\alpha$ antibodies and its use for treating IFN$\alpha$ related conditions.

**Figure 2**

**Description**

**FIELD OF INVENTION**

The present invention relates to an immunogenic vaccine and its use for treating IFNα related conditions such as systemic lupus erythematosus.

**BACKGROUND OF INVENTION**

The IFN type I family includes IFNα, IFNβ, IFNδ, IFN1, IFNκ, IFNτ, and IFNω̄. The predominant forms are IFNα, of which 13 closely related proteins are described in humans, and the single IFNβ. Despite the fact that different IFN type I forms may promote different biological responses, all IFN type I are structurally related (their genes lack introns and are located on the short arm of chromosome 9) and signal through the same receptor subunits (Van Boxel-Dezaire et al., Immunity 2006;25:361-372).

The interest on the relationship between IFN type I and autoimmune disorders is nowadays increasing, since the signs of its induction, the so-called interferon signature, have been recently reported in patients suffering from different autoimmune diseases (Baccala et al. Immunol Rev 2005;204:9-26). In fact, due to its immune-modulator effects, IFN type I seems to be involved in several pathogenic pathways of various autoimmune conditions.

The paradigm of IFN type I pathogenic relevance in autoimmunity is systemic lupus erythematosus (SLE). SLE is a chronic disease, characterized by a multi-organ involvement, due to a paradoxical damage of organs caused by autoantibodies directed to self-antigens. The etiology of SLE is complex, involving both genetic and environmental factors. The serum level of IFNα in SLE has been shown to correlate with the severity of the disease (Dall'era et al. Ann Rheum Dis 2005; 64:1692-7). Sjögren's syndrome (SS), also known as sicca syndrome, is a chronic, systemic, autoimmune condition which affects the exocrine glands, particularly the salivary and lachrymal glands. Elevated IFNα activity has also been observed in the serum of patients suffering from this disease. Finally, other conditions such as diabetes, rheumatoid arthritis, scleroderma, vasculitis and autoimmune thyroiditis have also been shown to be associated with high levels of IFNα.

Sedaghat et al. also recently suggested that type 1 IFN may play a role in CD4[+] T cells depletion in HIV[+] patients as they showed that type 1 IFN affect the steady state of normal CD4[+] T cells dynamics by shifting the balance towards Th1 effectors that are short lived cells instead of long-lived memory T cells (Sedaghat et al. J. Virol. 2008, 82 (4): 1870-1883). This was confirmed in Mandl et al., where it is suggested to diminish the IFNa production by plasmacytoid dendritic cells to ameliorate the pathological immune activation (Mandl et al. Nat. Med. 2008).

Moreover, administration of IFNα has been reported to exacerbate underlying disease in patients with psoriasis, autoimmune thyroiditis and multiple sclerosis and to induce an SLE like syndrome in patients without a previous history of autoimmune disease.

Therefore, there is a need for an agent that inhibits IFNα activity.

Passive immunization with monoclonal neutralizing antibodies is currently being tested in clinical trials with rontalizumab and sifalimumab for the treatment of SLE. However, said therapy presents the drawbacks of targeting only one subset of the 13 for IFNα and the use of passively administrated monoclonal antibodies can be limited by the induction of anti-drug antibodies. Said anti-drug antibodies may neutralize or otherwise compromise the clinical effect of the drugs and can also be associated with serious adverse events related to cross-reactivity with autologous proteins (De Groot et al. Trends. Immunol. 2007, 28(11)).

The present invention thus provides a method for inhibiting IFNα activity in vivo by administering a therapeutically effective amount of an immunogenic product that allows an active immunization which can break immunological B cell tolerance and generate high titers of polyclonal neutralizing antibodies against IFNα and its use for treating IFNα related conditions.

**SUMMARY**

One object of the invention is an immunogenic product comprising IFNα coupled to a carrier protein molecule for use in preventing or treating an IFNα related condition in a subject in need thereof, wherein the therapeutically effective amount of the immunogenic product to be administrated to the subject is more than 30 mcg of immunogenic product per administration.

In one embodiment of the invention, the administration of the therapeutically effective amount of the immunogenic product prevents the occurrence of symptoms of a disease linked to an over-production of IFNα.

In another embodiment of the invention, the administration of the therapeutically effective amount of the immunogenic product prevents the flare of a disease linked to an over-production of IFNα.

In another embodiment of the invention, the IFNα related conditions comprise systemic lupus erythematosus, rheumatoid arthritis, scleroderma, Sjögren syndrome, vasculitis, HIV, type I diabetes, autoimmune thyroiditis and myositis.

In another embodiment of the invention, the therapeutically effective amount of the immunogenic product to be administrated to the subject is from 35 mcg to 1000 mcg of immunogenic product per administration.

In another embodiment of the invention, the immunogenic product is administrated to the subject at least twice in a month.

In another embodiment of the invention, the immunogenic product is further administrated to the subject at least once every three months.

In another embodiment of the invention, the immunogenic product is further administrated to the subject when, in a serum sample obtained from the subject, the amount of anti- IFNα antibodies is undetectable.

In another embodiment of the invention, the immunogenic product is strongly inactivated, which means that the product shows less than 5% of antiviral activity in the conditions of TEST B.

In another embodiment of the invention, the immunogenic product is capable of neutralizing the antiviral activity of IFNα in the conditions of TEST C.

In another embodiment of the invention, the immunogenic product comprises at least one subtype of IFNα.

In another embodiment of the invention, the subtype of IFNα is IFNα 2b and the carrier protein molecule is KLH.

In another embodiment of the invention, the immunogenic product is a vaccine, preferably in the form of an emulsion.

Another object of the invention is a unit dosage form comprising more than 30 mcg of an immunogenic product comprising IFNα coupled to a carrier protein molecule as defined here above.

Another object of the invention is a medical device comprising more than 30 mcg of an immunogenic product comprising IFNα coupled to a carrier protein molecule as defined here above.

Another object of the invention is a kit comprising at least one vial containing more than 30 mcg of an immunogenic product comprising IFNα coupled to a carrier protein molecule as defined here above, at least one vial containing adjuvant, and means for contacting said solution to the adjuvant, and for emulsifying the mixture of the aqueous solution with the adjuvant.

## DEFINITIONS

As used herein, the term "interferon α" or "IFNα" refers to IFN alpha proteins encoded by a functional gene of the interferon alpha gene locus with 75% or greater sequence identity to IFN alpha 1 (Genbank number NP_076918 or protein encoded by Genbank number NM_024013). Examples of human IFN alpha subtypes include IFN alpha 1 (Genbank number NP_076918), alpha 2a (Genbank number ITF_A), alpha 2b (Genbank number AAP20099), alpha 4 (Genbank number NP_066546), alpha 5 (Genbank number P01569), alpha 6 (P05013), alpha 7 (Genbank number P01567), alpha 8 (Genbank number P32881), alpha 10 (Genbank number P01566), alpha 14 (Genbank number P01570), alpha 16 (Genbank number NP_002164), alpha 17 (Genbank number P01571) and alpha 21(Genbank number NP_002166). Examples of nonhuman mammal IFNa subtype may be found in Genbank as well known by the person skilled in the art (for review see Pestka et al Immunological reviews 2004, 202:8-32).

As used herein, the term "immune response" refers to the action, for example of lymphocytes, antigen presenting cells, phagocytic cells and macromolecules produced by the above cells or the liver (including antibodies, cytokines and complement).

As used herein, an antibody that "inhibits the biological activity" or "neutralizes the biological activity" of IFNα is intended to refer to an antibody that inhibits the activity of that cytokine by at least 10%, 20%, 30%, 40%, 50%, 60%, 70%, or 80% or more, as compared to the level of activity of the cytokine in the absence of the antibody, for example by using a functional assay such as those described in the Examples.

As used herein, the term "carrier protein molecule" refers to a protein or a peptide of at least 15 amino acids long which, when partially covalently being associated to the IFNα molecule for forming heterocomplexes, allows for a large number of antigens of IFNα to be presented to the B lymphocytes.

As used herein, the term "subject" includes any human or nonhuman mammals such as primates, dogs, cats, horses, sheep...

As used herein, the term "patient" refers to a subject that is affected by an IFNα related condition.

As used herein, the term "effective amount" refers to an amount sufficient to cause a beneficial or desired clinical result (e.g. improvement in clinical condition).

As used herein, the term "treatment" refers to clinical intervention in an attempt to alter the natural course of a disease of the subject or patient to be treated, and may be performed either for prophylaxis or during the course of clinical pathology. Desirable effects include, but are not limited to, preventing occurrence or recurrence of disease, alleviating symptoms, suppressing, diminishing or inhibiting any direct or indirect pathological consequences of the disease, lowering the rate of disease progression, ameliorating or palliating the disease state, and causing remission, maintaining remission state or improved prognosis.

## DETAILED DESCRIPTION

Although regulators of IFNα occur naturally in the body, their capacity to regulate the cytokine levels in diseases such as SLE and SS appears to be insufficient. The aim of the anti- IFNα therapeutic immunization of the invention is to raise antibody levels against the cytokine, while enhancing their affinity and neutralizing activity, resulting in the reduction of the excess cytokine and inhibiting its pathogenic effects, without interfering with other metabolic and physiological processes.

One object of the present invention is a method for treating an IFNα related condition in a subject in need thereof, comprising administering to the subject a therapeutically effective amount of an immunogenic product comprising IFNα coupled to a carrier protein molecule, wherein said therapeutically effective amount is more than 30 mcg (μg) of immunogenic product per administration.

In one embodiment of the invention, the therapeutically effective amount of the immunogenic product per administration is from more than 30 mcg to 1000 mcg. In another embodiment of the invention, the therapeutically effective amount of the immunogenic product per administration is from more than 30 mcg to 750 mcg. In another embodiment of the invention, the therapeutically effective amount of the immunogenic product per administration is from more than 30 mcg to 500 mcg. In another embodiment of the invention, the therapeutically effective amount of the immunogenic product per administration is from more than 30 mcg to 450 mcg. In another embodiment of the invention, the therapeutically effective amount of the immunogenic product per administration is from more than 30 mcg to 400 mcg. In another embodiment of the invention, the therapeutically effective amount of the immunogenic product per administration is from more than 30 mcg to 350 mcg. In another embodiment of the invention, the therapeutically effective amount of the immunogenic product per administration is from more than 30 mcg to 300 mcg. In another embodiment of the invention, the therapeutically effective amount of the immunogenic product per administration is from more than 30 mcg to 250 mcg. In another embodiment of the invention, the therapeutically effective amount of the immunogenic product per administration is from more than 30 mcg to 200 mcg. In another embodiment of the invention, the therapeutically effective amount of the immunogenic product per administration is from more than 30 mcg to 150 mcg. In another embodiment of the invention, the therapeutically effective amount of the immunogenic product per administration is from more than 30 mcg to 100 mcg.

In another embodiment of the invention, the therapeutically effective amount of the immunogenic product per administration is from 35 mcg to 1000 mcg. In another embodiment of the invention, the therapeutically effective amount of the immunogenic product per administration is from 35 mcg to 750 mcg. In another embodiment of the invention, the therapeutically effective amount of the immunogenic product per administration is from 35 mcg to 500 mcg. In another embodiment of the invention, the therapeutically effective amount of the immunogenic product per administration is from 35 mcg to 450 mcg. In another embodiment of the invention, the therapeutically effective amount of the immunogenic product per administration is from 35 mcg to 400 mcg. In another embodiment of the invention, the therapeutically effective amount of the immunogenic product per administration is from 35 mcg to 350 mcg. In another embodiment of the invention, the therapeutically effective amount of the immunogenic product per administration is from 35 mcg to 300 mcg. In another embodiment of the invention, the therapeutically effective amount of the immunogenic product per administration is from 35 mcg to 250 mcg. In another embodiment of the invention, the therapeutically effective amount of the immunogenic product per administration is from 35 mcg to 200 mcg. In another embodiment of the invention, the therapeutically effective amount of the immunogenic product per administration is from 35 mcg to 150 mcg. In another embodiment of the invention, the therapeutically effective amount of the immunogenic product per administration is from 35 mcg to 100 mcg.

In another embodiment of the invention, the therapeutically effective amount of the immunogenic product per administration is from 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390 mcg to 400 mcg.

In another embodiment of the invention, the therapeutically effective amount of the immunogenic product per administration is from 60 mcg to 240 mcg. In another embodiment of the invention, the therapeutically effective amount of the immunogenic product per administration is 60 mcg. In another embodiment of the invention, the therapeutically effective amount of the immunogenic product per administration is 120 mcg. In another embodiment of the invention, the therapeutically effective amount of the immunogenic product per administration is 240 mcg.

In one embodiment of the invention, the subject to be treated is administrated at least twice in a month with the therapeutically effective amount of immunogenic product as described here above.

In another embodiment of the invention, the subject to be treated is administrated two times in 1 month with the therapeutically effective amount of immunogenic product as described here above. In this embodiment, the subject may be administrated once at day 0 and the second time between day 7 and day 28. In another embodiment, the subject may be administrated once at day 0 and the second time between day 7 and day 21. In one embodiment, the subject is administrated once at day 0 and the second time at day 28.

In another embodiment of the invention, the subject to be treated is administrated three times in 1 month with the therapeutically effective amount of immunogenic product as described here above. In this embodiment, the subject to be treated may be administrated once at day 0, the second time between day 7 and day 14 and the third time between day 21 and day 28. In one embodiment, the subject is administrated once at day 0, the second time at day 7 and the third time at day 28.

In another embodiment of the invention, the subject to be treated may be further administrated once every three months with the therapeutically effective amount of the immunogenic product as described here above.

In another embodiment of the invention, the subject to be treated may be further administrated once every six months with the therapeutically effective amount of the immunogenic product as described here above.

In another embodiment of the invention, the subject to be treated may be further administrated once a year with the therapeutically effective amount of the immunogenic product as described here above.

In another embodiment of the invention, the subject to be treated may be further administrated once every 5 years with the therapeutically effective amount of the immunogenic product as described here above.

In another embodiment of the invention, the subject to be treated may be further administrated once every 10 years with the therapeutically effective amount of the immunogenic product as described here above.

In another embodiment of the invention, the subject to be treated may be further administrated with the therapeutically effective amount of the immunogenic product as described here above when the amount of antibodies against IFN$\alpha$ is undetectable in a serum sample obtained from the subject.

Quantification of the amount of antibodies against IFN$\alpha$ in a serum sample may be carried out by conventional methods known in the art, such as an ELISA anti-IFN.

One example of carrying out such method is the following:

- coating a 96 wells plate with 100 ng of the subtype of IFN$\alpha$ used for preparing the immunogenic product such as IFN$\alpha$ -2b and incubate the plate overnight at 2°C-8°C,
- blocking the plate with a blocking buffer during 90 min at 37°C,
- incubating the plate with the serum sample and pool of naive sample during 90 min at 37°C: the serum sample is typically diluted in a two fold dilution series starting from dilution 200x to at least 8 dilutions,
- incubating the plate with the labeled secondary antibody such as a goat antihuman immunoglobulin conjugated to HRP,
- developing the complex with an o-phenylenediamine dihydrochloride (OPD) substrate solution. After stopping the enzymatic reaction, the intensity of the resulting color is determined by spectrophotometric methods at 492 nm.

The anti-IFN titer for each sample is expressed as the minimal dilution for which the mean OD value is higher than the cut-off value:

$$\text{Cut-off value= Mean OD of the pool of naive serum x 2.08}$$

where the N cut-off value is equal to 2.08.

Then, the anti-IFN titer for each sample will be expressed as the minimal dilution for which the mean OD value is higher than the cut-off value. The first dilution being 200, patients are considered negative if their OD at 1/200 is inferior to the cut-off value (Mire-Sluis et al. 2004 J. Immunol Meth. 289: 1-16).

In one embodiment of the invention, the subject to be treated is suffering from an IFN$\alpha$ related condition.

In another embodiment of the invention, the subject to be treated presents undetectable amount of anti- IFN$\alpha$ antibodies in the serum.

### [Mechanism of action]

The present invention also relates to an immunogenic product that is useful for inducing an immune response in a mammal to whom said immunogenic product is administered, including a humoral immune response wherein antibodies that neutralize the immmunosuppressive, apoptotic or angiogenic properties of the endogenous cytokine IFN$\alpha$.

In one embodiment of the invention, the immunogenic product is an inactivated but immunogenic cytokine derivative of IFN$\alpha$ chemically coupled to a T-helper stimulating foreign carrier protein such as for example KLH. Said immunogenic product has the ability to disrupt B cell but not T cell tolerance to IFN$\alpha$. Helper T cell tolerance against self is circumvented by linking IFN$\alpha$ to the foreign carrier protein.

B cells specific for IFN$\alpha$ are activated following antigen binding and endocytose the immunogenic product and carrier specific peptides are presented via the Major Histocompatibility Complex (MHC) class II molecules. This activation signal is not sufficient to induce B cell differentiation in the case of a T dependent antigen but because B cells process the self and the carrier antigens, T cell help can be given by T cells specific for the self or the carrier protein. Since T cell selection is very stringent, there is no specific T cell activation for the self antigen.

Dendritic cells (DC) can also take up the self antigen and the carrier molecule and present carrier specific peptides via their MHC class II molecules. DCs are thus able to activate naive T helper cells specific for the carrier. The T helper cells are in turn able to provide carrier-specific T helper cells to B cells specific for the self antigen and to present carrier peptides on their MHC class II molecules.

T helper cells specific for the carrier interact with B cells specific for the self antigen, eliciting a normal antibody response against the self antigen, i.e. v.

The immunogenic product is mainly used in vaccine compositions for preventing or treating a disease linked to an over-production of IFNα.

More specifically, this invention relates to a method for preventing or treating a disease linked to an over-production of IFNα comprising a step of administering to the subject, a therapeutically effective amount of the immunogenic product of the invention.

This invention also relates to a method for preventing or treating a disease linked to an over-production of IFNα comprising the administration of a therapeutically effective amount of the immunogenic product, wherein the administration of the immunogenic product prevents the occurrence of symptoms of the disease.

The invention also relates to a method for preventing or treating a disease linked to an over-production of IFNα comprising the administration of a therapeutically effective amount of the immunogenic product, wherein the administration of the immunogenic product prevents the flare of the disease.

The invention also relates to a method for preventing or treating a disease linked to an over-production of IFNα comprising the administration of a therapeutically effective amount of the immunogenic product, wherein the administration of the immunogenic product induces the production of antibodies that neutralize the activity of endogeneous IFNα.

The invention also relates to a method for preventing or treating a disease linked to an over-production of IFNα comprising the administration of a therapeutically effective amount of the immunogenic product, wherein the administration of the immunogenic product induces the neutralization of the activity of endogeneous IFNα.

Examples of disease linked to an over-production of IFNα include, but are not limited to systemic lupus erythematosus, rheumatoid arthritis, scleroderma, Sjögren syndrome, vasculitis, HIV, type I diabetes, autoimmune thyroiditis and myositis.

A further object of the invention consists of a method for inducing the production of antibodies that neutralize the activity of endogeneous IFNα in a subject, comprising a step of administering to said subject a therapeutically effective amount of the immunogenic product.

## [The immunogenic product]

The immunogenic product as used in the invention comprises IFNα coupled to a carrier protein molecule such as KLH, wherein the immunogenic product is inactivated.

The immunogenic product as used in the invention is a complex between at least one recombinant IFNα subtype and at least one carrier protein molecule such as for example KLH obtained by conjugation with glutaraldehyde and subsequent inactivation with formaldehyde.

In one embodiment of the invention, the carrier protein molecule may be any carrier molecule conventionally used in immunology such as KLH (Keyhole limpet hemocyanin), ovalbumin, bovine serum albumin (BSA), toxoid tetanos, toxoid diphteric B cholera toxin....In one preferred embodiment, said carrier is KLH. Preferably, the KLH starting product consists of a highly purified KLH extracted from the lymph of the marine gastropod mollusk *Megathura cremulata.* Naturally produced KLH generally consists of a di-decamer structure which is a non covalent tubular assembly of 20 subunits.

In another embodiment of the invention, the recombinant IFNα subtype may be any subtype among IFN alpha 1, alpha 2a, alpha 2b, alpha 4, alpha 5, alpha 6, alpha 7, alpha 8, alpha 10, alpha 14, alpha 16, alpha 17 and alpha 21.

Recombinant IFNα subtypes may be obtained by conventional methods known in the art using the sequences from Genbank as described here above. For example, production of the recombinant IFNα subtype may be carried out by culturing cells containing an expression vector comprising the gene of the IFNα subtype and then harvesting the inclusion bodies and finally purifying the IFNα subtype.

In one embodiment of the invention, the recombinant IFNα subtype is the IFNα 2b subtype.

In one embodiment of the invention, the immunogenic product comprises at least the IFNα 2b subtype.

In one embodiment of the invention, the recombinant IFNα subtype is in a liquid solution, preferably a buffer solution having a pH ranging from 3.5 to 7.8.

In one embodiment, when the subject to be treated is a human, the recombinant IFNα used is human.

In one embodiment of the invention, the immunogenic product comprises IFNα coupled to a carrier protein molecule such as for example KLH, wherein said immunogenic product is recognized by an anti- IFNα antibody.

The recognition of the immunogenic product by an anti- IFNα antibody may be carried out by conventional methods known in the art such as a sandwich ELISA anti-IFNα/carrier protein. The ELISA (TEST D) are developed by any colorimetric means known in the art such as for example using detection antibody labelled with biotin, a poly-streptavidin HRP amplification system and an o-phenylenediamine dihydrochloride substrate solution.

One example of said method is the following:

- coating a plate with the capture antibody, such as for example a rabbit polyclonal anti-KLH antibody,
- blocking the plate with a blocking buffer (such as casein 2% in PBS for example) during 90 min at 37°C,
- incubating during 90 min at 37°C the plate with a dilution series of the immunogenic product from 250 ng/ml to 8 two fold dilutions or with negative controls such as KLH and IFNα,
- incubating 90 min at 37°C the plate with the detection antibody such as for example a biotinylated anti-IFNa antibody,
- incubating the plate with streptavidin-HRP during 30 min at 37°C and developing the complex with an o-phenylenediamine dihydrochloride (OPD) substrate solution furing 30 min. After stopping the enzymatic reaction, the intensity of the resulting color is determined by spectrophotometric methods at 490 nm.

When optical density of wells containing the immunogenic product is at least 10 times superior to the optical density of wells containing the negative control, the person skilled in the art considers that the immunogenic product is recognized by an anti- IFNα antibody and that IFNα in the immunogenic product is coupled to the KLH.

In another embodiment of the invention, the immunogenic product comprises IFNα coupled to a carrier protein molecule such as for example KLH, wherein said immunogenic product is strongly immunogenic, which means that the product is capable of inducing antibodies anti-IFNα in vivo in the conditions of hereunder tested TEST A.

Test A is carried out according to the following method:

0.3 to 10 µg of total proteins (as determined by a Bradford protein assay) of the immunogenic product is injected in Balb/c mice of 6-8 weeks twice in 30 days, preferably at day 0 and day 21. A serum sample is obtained before immunization (pre-immune serum sample) and between day 30 and day 40 (test serum sample), preferably at day 31. An ELISA anti-IFNα is carried out as explained here above.

Briefly, a 96 wells plate is coated with 100 ng of the subtype of IFNα used for preparing the immunogenic product such as IFNα -2b and incubated overnight at 2°C-8°C. The plate is then blocked with a blocking buffer during 90 min at 37°C. 100 µl of pre-immune sample at dilution 1/2500 and a dilution series from 1/2500 up to 8 two fold dilutions of the serum samples (pre-immune and test) are added to the wells. An anti-mouse immunoglobulins labeled secondary antibody such as an HRP conjugated antibody is finally added to the wells and the ELISA is developed using any colorimetric means known in the art such as for example an o-phenylenediamine dihydrochloride substrate solution.

When optical density of wells containing the test serum sample is at least 2 times superior to the optical density of wells containing the pre-immune serum sample, the person skilled in the art considers that the immunogenic product is immunogenic, which means that it had induced anti-IFNα antibodies in vivo.

In another embodiment of the invention, the immunogenic product comprises IFNα coupled to a carrier protein molecule such as for example KLH, wherein the IFNα is strongly inactivated, which means that the product shows less than 5%, preferably less than 1% of antiviral activity of IFNα in the conditions of hereunder cited TEST B.

This assay is based on the protective effect of IFNα on the cytopathic effect (CPE) of Vesicular Stomatitis Virus (VSV) on Madin-Darby Bovine Kidney (MDBK) cells. This assay may also be carried out using Hep-2C or A549 human cells and EMCV virus. Test B is carried out according to the following method:

The immunogenic product and the recombinant IFNα subtype used for preparing the immunogenic product (positive control) are diluted at at least 500 ng/ml and at least 1000 U/ml respectively in Basal medium (RPMI supplemented with 2 mM glutamine, 1 mM sodium pyruvate, 1 mM Hepes). 50 µl of the immunogenic product and the positive control are plated in a 96 wells plate and diluted in a series of two fold dilutions in the Basal medium. $2 \cdot 10^4$ MDBK cells are added in each well in 50 µl of Cell medium (RPMI supplemented with 4% FBS, 2 mM glutamine, 1 mM sodium pyruvate and 1 mM Hepes) and the plate is incubated overnight at 37°C, 5% $CO_2$. The virus is then diluted in Basal medium to at least 20000 $TCID_{50}$ (Tissue Culture Infection Dose 50: 10 times the dilution to kill 50% of infected cells). The plate is emptied and 100 µl of the diluted virus is added. The plate is then incubated overnight at 37°C, 5% $CO_2$.

At the end of the culture, viability of the MDBK cells is assessed using methods well-known in the art. One example of said methods is the following: 20 µl/well of a solution of MTS/PMS (100 µl MTS/5 µl PMS; Promega G5430) are added to the wells and the plate is incubated for another 4h at 37°C 5% CO2. The plate is then read at 490 nm on a spectrophotometer.

The percentage of antiviral activity is calculated as following:

$$\% \text{ antiviral activity} = [(OD_{product} - OD_{virus}) / \text{mean } OD_{cells} - OD_{virus})] * 100$$

$OD_{product}$ stands for the optical density of well with the immunogenic product or with the positive control (IFNα subtype).

$OD_{virus}$ stands for the optical density of control well with the virus only.

$OD_{cells}$ stands for the optical density of control well with IFNα and virus.

The $EC_{50}$ value, corresponding to the amount of immunogenic product resulting in 50% inhibition of virus-mediated mortality, is determined by interpolating the $EC_{50}$ value onto the x axis on a viability/concentration graph.

Comparing the $EC_{50}$ of the immunogenic product and the $EC_{50}$ of the positive control (the recombinant IFNα subtype used for preparing the immunogenic product) allows determining whether the immunogenic product shows less than 5%, preferably less than 1% of antiviral activity.

An Inactivation Factor $EC_{50\ product}$ / $EC_{50\ IFN\alpha}$ can be calculated: when the immunogenic product shows less than 5%, preferably less than 1% of antiviral activity, the Inactivation Factor is more than 20, preferably more than 100.

In another embodiment of the invention, the immunogenic product comprises IFNα coupled to a carrier protein molecule such as for example KLH, wherein the immunogenic product is capable of neutralizing the antiviral activity of IFNα in the conditions of hereunder cited TEST C.

According to the invention, this assay is performed to evaluate the neutralizing capacity of the serum obtained from mice immunized with the immunogenic product. The neutralizing capacity may be assessed by evaluating the cell viability in presence of the vesicular stomatitis virus replicating in MDBK cells. This assay may also be carried out using Hep-2C human cells and EMCV virus.

Test C is carried out according to the following method:

0.3 to 10 μg of total proteins (as determined by a Bradford protein assay) of the immunogenic product is injected in Balb/c mice of 6-8 weeks twice in 30 days, preferably at day 0 and day 21. A serum sample is obtained before immunization (pre-immune serum sample) and between day 30 and day 40 (test serum sample), preferably at day 31.

25 μl of pre-immune and test serum samples are plated in a 96-well plate at a dilution of 1/200 up to 8 dilutions from 1/200. The positive control (polyclonal anti-IFNα from PBL, Piscataway, NJ, ref.31100-1) is typically diluted to be able to neutralize IFNα activity from 3125 UI/well to 100 UI/well in Basal medium (RPMI supplemented with 2 mM glutamine, 1 mM sodium pyruvate and 1 mM hepes) and 25 μl were also plated in the plate.

25 U/well (final concentration) in 25 μl of basal medium of IFNα is added to each well and the plate is incubated for 60 min at room temperature.

20000 MDBK cells in Assay medium (RPMI supplemented with 4% FBS, 2 mM glutamine, 1 mM sodium pyruvate, 1 mM hepes) are added to each well and the plate is incubated overnight at 37°C, 5% $CO_2$.

The virus is diluted to at least 20000 $TCID_{50}$ (10 times the dilution to kill 50% of infected cells) in virus medium (RPMI supplemented with 2 mM glutamine, 1 mM sodium pyruvate, 1 mM hepes). The plate is emptied and 100 μl of virus is added to each well before incubation for 24h at 37°C, 5% $CO_2$.

At the end of the culture, viability of the MBDK cells is assessed using methods well-known in the art. One example of said methods is the following: 20 μl/well of a solution of MTS/PMS (100 μl MTS/5 μl PMS; Promega G5430) are added to the wells and the plate is incubated for another 4h at 37°C 5% CO2. The plate is then read at 490 nm on a spectrophotometer.

The relative cell viability is calculated as following:

$$\% = [(OD_{sample} - OD_{virus}) / OD_{IFN+virus})] * 100$$

$OD_{sample}$ stands for the optical density of well with the serum obtained from the mouse immunized with the immunogenic product or with the positive control (polyclonal anti-IFN antibody).

$OD_{virus}$ stands for the optical density of control well with the virus only.

$OD_{IFN+virus}$ stands for the optical density of control well with IFNα and virus.

The $NC_{50}$ value, corresponding to the dilution of serum resulting in 50% neutralization of virus-mediated mortality expressed as a dilution factor or neutralizing unit/ml , is determined by interpolating the $NC_{50}$ value onto the x axis on a viability/concentration graph.

In TEST C, a result showing that the serum obtained from the mouse immunized with the immunogenic product does not protect the MBDK cells from mortality means that the immunogenic product has the capacity to induce antibodies directed against IFNα that neutralize its antiviral activity.

In one embodiment, the immunogenic product comprises IFNα coupled to a carrier protein molecule such as for example KLH, wherein the immunogenic product is capable of neutralizing at least 50% of the antiviral activity of IFNα in the conditions of TEST C. In said embodiment, the $NC_{50}$ can be calculated. If the dilution of serum is not capable of neutralizing at least 50% of the antiviral activity of IFNα in the conditions of TEST C, the $NC_{50}$ of the product cannot be calculated.

**[Method for obtaining the immunogenic product]**

In one embodiment of the invention, the IFNα kinoid is obtained according to the following method:

a) mixing together the at least one recombinant human IFNα subtype and the at least one carrier protein molecule with glutaraldehyde and blocking the reaction by adding a quenching compound selected from (i) a reducing agent and (ii) an amino acid selected from the group consisting of lysine and glycine and mixture thereof,
b) removing compounds having a molecular weight of less than 10 kDa, or of less than 8 kDa
c) adding formaldehyde;
d) blocking the reaction with formaldehyde by adding a quenching compound selected from (i) a reducing agent and (ii) an amino acid selected from the group consisting of lysine and glycine and mixture thereof,
e) collecting the said immunogenic product.

In one embodiment of step a), IFNα and the carrier protein molecule such as for example KLH are firstly mixed together in the appropriate amounts, before adding glutaraldehyde.
In one embodiment, IFN α and KLH are mixed at step a) at a IFNα:subunitKLH molar ratio ranging from 10:1 to 40:1. In another embodiment, IFN α and KLH are mixed at step a) at a IFN α:subunitKLH molar ratio ranging from 15:1 to 25:1. In another embodiment, IFN α and KLH are mixed at step a) at a IFN α:subunitKLH molar ratio ranging from 20:1 to 25:1.
In one embodiment of step a), glutaraldehyde is used at a final concentration in the reaction mixture ranging from 1 mM to 250 mM, preferably from 20 mM to 30 mM, more preferably from 22.5 mM to 25 mM. In one embodiment of step a), glutaraldehyde is incubated with IFN α and KLH for a period of time ranging from 15 min to 120 min, preferably about 30, 35, 40, 45, 50, 60, 70, 80, 90 minutes. In one embodiment, glutaraldehyde is added at 22.5 mM during about 45 minutes. Advantageously, step a) of incubation with glutaraldehyde is performed at a temperature ranging from 18°C to 37°C, preferably from 18°C to 27°C.
According to an embodiment, the reaction with glutaraldehyde (step a) is stopped prior to removing compounds having a molecular weight of less than 10 kDa, (step b) by adding a quenching compound, preferably a quenching compound that is selected from (i) a reducing agent and (ii) an amino acid selected from the group consisting of lysine and glycine and mixture thereof.
The reducing agent may consist of any one of the reducing agents known in the art which, due to their reducing properties, have the ability to reduce the remaining imine groupments generated during aldehyde treatment. The reducing agent may be selected from the group consisting of sodium borohydride, sodium cyanoborohydride.
According to an embodiment, in the embodiments wherein the said quenching compound is an amino acid, the said amino acid consists of glycine. In some embodiments of step b) where glycine and/or lysine are used for blocking the reaction with glutaraldehyde, the selected amino acid is used at a final concentration in the reaction mixture ranging from 0.01 M to 1 M, preferably from 0.05 M to 0.5 M, and most preferably from 0.08 M to 0.2 M, e.g. at 0.1 M as shown in the examples herein. In an embodiment, incubation with the quenching compound is performed for a period of time ranging from 1 minute to 120 minutes, preferably from 5 minutes to 60 minutes, e.g. for 30 minutes as shown in the examples herein. In another embodiment, this step is performed at a temperature ranging from 18°C to 30°C, preferably from 18°C to 25°C.
At step b), the small compounds of less than 10 kDa that are present in the reaction mixture are removed. These small compounds encompass mainly the excess glutaraldehyde and the excess quenching compound molecules that have not reacted with IFN α nor KLH. Step b) may be performed according to any known technique which allows removing compounds of less than 10 kDa, which techniques include dialysis with a dialysis membrane having a cut-off of 10kDa or filtration using a filtration membrane having a cut-off of 10 kDa. Illustratively, step b) may consist of a step of tangential flow filtration using a filtration membrane having a cut-off of 10 kDa, as it is shown in the examples herein. The filtration retentate, which is devoid of the undesirable small compounds, is collected at the end of step b). If desired, step b) may comprise a preliminary step of removing the eventual compound aggregates present in the reaction mixture obtained at the end of step b). The said preliminary step may consist of a conventional filtration step for removing aggregates eventually present in suspension in a liquid solution, e.g. a filtration step using an appropriate filtration membrane, e.g. a filtration membrane having a pore size of 0.2 μm.
In one embodiment of step c) of the method, formaldehyde is added at a final concentration from 6 mM to 650 mM, preferably from 25 mM to 250 mM. In one embodiment of step c) of the method, formaldehyde is added for a period of time from 1h to 336 hours, preferably from 1h to 144 hours. In one embodiment, formaldehyde is applied at a final concentration of 50 to 100 mM, preferably 66 mM during 20 to 50 hours, preferably 40 hours.

At step c), incubation with formaldehyde is performed preferably at a temperature ranging from 30°C to 40°C, e.g. at 37°C as it is shown in the examples herein.

At step d) of the method, the reaction with formaldehyde is stopped by adding a quenching compound, preferably a quenching compound that is selected from (i) a reducing agent and (ii) an amino acid selected from the group consisting of lysine and glycine.

The reducing agent may consist in any one of the reducing agents known in the art which, due to their reducing properties, reduce the remaining imine groupements generated during aldehyde treatment. The reducing agent may be selected from the group consisting of sodium borohydride, sodium cyanoborohydride. According to an embodiment, in the embodiments wherein the said quenching compound is an amino acid, the said amino acid consists of glycine. In some embodiments of step b) where glycine and/or lysine are used for blocking the reaction with formaldehyde, the selected amino acid is used at a final concentration in the reaction mixture ranging from 0.01 M to 1.5 M, preferably from 0.05 M to 1 M, and most preferably from 0.1 M to 0.2 M, e.g. at 0.1 M as shown in the examples herein. In an embodiment, incubation with the quenching compound is performed for a period of time ranging from 5 minutes to 120 minutes, preferably from 10 minutes to 60 minutes, e.g. for 30 minutes as shown in the examples herein. In another embodiment, this step is performed at a temperature ranging from 18°C to 30°C, preferably from 18°C to 25°C.

According to one embodiment of the method, just prior to collecting at step e), removal of substances having a molecular weight of less than 100 kDa may be performed by the skilled artisan by any technique known in the art for removing substances having a molecular weight of more than 100 kDa from a liquid solution. In a first embodiment, the technique used is a filtration step that is performed by using a filtration membrane having a cut-off value of at least 100 kDa, which encompasses an ultrafiltration step or a tangential filtration step. In a second embodiment, the technique used consists of a tangential filtration step using a filtration membrane having a cut-off value of at least 100 kDa. In another embodiment, just prior to collecting at step e), removal of substances having a molecular weight of less than 300 kDa may be performed by using a filtration membrane having a cut-off value of at least 300 kDa.

### [Composition, Emulsion and vaccine containing such emulsion]

This invention relates to a composition comprising the immunogenic product as described here above. This invention also relates to a formulation of the product of the invention, wherein the product is within an emulsion. Advantageously, the vaccine composition of the invention comprises or consists of said emulsion. Such emulsion comprises the immunogenic product of the invention, an oil and a surfactant or a mixture of at least one oil and at least one surfactant. Preferably, the oil or the mixture oil/surfactant is a pharmaceutically acceptable excipient. More preferably, the mixture of oil and surfactant is an adjuvant, even more preferably an immunoadjuvant. Preferred adjuvant is ISA 51. The emulsion of the invention may be a water-in-oil emulsion or an oil-in-water emulsion.

In another embodiment, the amount of the immunogenic product according to the invention is of more than 0.01% (w/w) and less than 1% (w/w) of the total weight of the said emulsion.

### [Adjuvants]

The emulsion or the vaccine composition of the invention may comprise adjuvant, especially immunoadjuvants. In an embodiment, the amount of adjuvant ranges from 0.00001% (w/w) to 1%, preferably 0.0001 to 0.1%, more preferably from 0.001 to 0.01% (w/w) of the total weight of the vaccine composition.

Any suitable adjuvant known by the skilled artisan may be used in the vaccine composition above, including oil-based adjuvants such as for example Freund's Incomplete Adjuvant, mycolate-based adjuvants (e.g., trehalose dimycolate), bacterial lipopolysaccharide (LPS), peptidoglycans (i.e., mureins, mucopeptides, or glycoproteins such as N-Opaca, muramyl dipeptide [MDP], or MDP analogs), MPL (monophosphoryl lipid A), proteoglycans (e.g., extracted from Klebsiella pneumoniae), streptococcal preparations (e.g., OK432), Biostim.TM.(e.g., 01 K2), the "Iscoms" of EP 109 942, EP 180 564 and EP 231 039, aluminum hydroxide, saponin, DEAE-dextran, neutral oils (such as miglyol), vegetable oils (such as arachid oil), liposomes, Pluronic.RTM. polyols, the Ribi adjuvant system (see, for example GB-A-2 189 141), or interleukins, particularly those that stimulate cell mediated immunity. An alternative adjuvant consisting of extracts of Amycolata, a bacterial genus in the order Actinomycetales, has been described in U.S. Pat. No. 4,877,612. Additionally, proprietary adjuvant mixtures are commercially available. The adjuvant used will depend, in part, on the recipient organism. The amount of adjuvant to administer will depend on the type and size of animal. Optimal dosages may be readily determined by routine methods.

Oil adjuvants suitable for use in water-in-oil emulsions may include mineral oils and/or metabolizable oils. Mineral oils may be selected from Bayol®., Marcol.®. and Drakeol, including Drakeol® 6VR (SEPPIC, France). ®.

Metabolisable oils may be selected from SP oil (hereinafter described), Emulsigen (MPV Laboratories, Ralston, NZ), Montanide 264,266,26 (Seppic SA, Paris, France), as well as vegetable oils, such as peanut oil and soybean oil, animal oils such as the fish oils squalane and squalene, and tocopherol and its derivatives.

In addition, the adjuvant may include one or more wetting or dispersing agents in amounts of about 0.1 to 25%, more preferably about 1 to 10%, and even more preferably about 1 to 3% by volume of the adjuvant. Particularly preferred as wetting or dispersing agents are non-ionic surfactants. Useful non-ionic surfactants include polyoxyethylene/polyoxypropylene block copolymers, especially those marketed under the trademark Pluronic®. and available from BASF Corporation (Mt. Olive, N.J.). Other useful nonionic surfactants include polyoxyethylene esters such as polyoxyethylene sorbitan monooleate, available under the trademark Tween 80®, or mannide monooleate. It may be desirable to include more than one, e.g. at least two, wetting or dispersing agents in the adjuvant as part of the vaccine composition of the invention.

Suitable adjuvants may include but are not limited to surfactants known by one skilled in the art, such as for example hexadecylamine, octadecylamine, lysolecithin, dimethyldioctadecylammonium bromide, N,N-dioctadecyl-N'-N-bis(2- hydroxyethylpropane di-amine), methoxyhexadecyl-glycerol, and pluronic polyols; polanions, e.g., pyran, dextran sulfate, poly IC, polyacrylic acid, carbopol; peptides, e.g., muramyl dipeptide, aimethylglycine, tuftsin, oil emulsions, alum, and mixtures thereof. Other potential adjuvants include the B peptide subunits of E. coli heat labile toxin or of the cholera toxin. McGhee, J. R., et al., "On vaccine development," Sem. Hematol., 30:3-15 (1993).

### *[Further surfactants]*

In the embodiments of a vaccine composition according to the invention comprising an emulsion, the vaccine composition preferably contains, in addition to the combination of the immunogenic product and the one or more oily immunoadjuvant substances, also one or more surfactant agents. Illustrative embodiments of surfactive agents include mannide monoleate such as Montanide® 80 marketed by Arlacel (SEPPIC, France).

In an embodiment, the amount of surfactant agent ranges from 0.00001% (w/w) to 1%, preferably 0.0001 to 0.1%, more preferably from 0,001 to 0.01% (w/w) of the total weight of the vaccine composition.

### *[Lyophilized products]*

According to an embodiment and for storage purposes, the product or the vaccine composition of the invention may be lyophilized. Vaccine compositions may thus be presented in a freeze-dried (lyophilized) form. In said embodiment, the immunogenic product according to the invention is combined with one or more lyophilisation auxiliary substances. Various lyophilisation auxiliary substances are well known by the one skilled in the art. Lyophilization of auxiliary substances encompasses sugars like lactose and mannitol.

In such embodiment where the vaccine composition consists of a lyophilised composition for use as a liquid emulsion comprising a surfactant agent, the vaccine composition preferably comprises an amount of the immunogenic product according to the invention of more than 0.1% (w/w) and less than 10% (w:/w) of the total weight of the said vaccine composition.

### *[Stabilizers]*

In some embodiments, the vaccine may be mixed with stabilizers, e.g. to protect degradation-prone proteins from being degraded, to enhance the shelf-life of the vaccine, or to improve freeze-drying efficiency. Useful stabilisers are i.a, SPGA (Bovarnik et al; J. Bacteriology 59: 509 (1950)), carbohydrates e.g. sorbitol, mannitol, trehalose, starch, sucrose, dextran or glucose, proteins such as albumin or casein or degradation products thereof, and buffers, such as alkali metal phosphates.

### *[Administration route]*

The vaccine compositions according to the invention may be administered to the subject to be immunized by any conventional method including, by injectable, e.g. intradermal, intramuscular, intraperitoneal, or subcutaneous injection; or by topical, such as for example by transdermal delivery. The treatment may consist of a single dose or a plurality of doses over a period of time.

### *[Dosage form]*

The forms suitable for injectable use may include sterile solutions or dispersions and sterile powders for the extemporaneous preparation of sterile injectable solutions or dispersions. The prevention against contamination by microorganisms can be brought about by adding in the vaccine composition various antibacterial and antifungal agents, for example, parabens, chlorobutanol, phenol, sorbic acid, thimerosal and the like. In many cases, it may be preferable to include isotonic agents, for example, sugars or sodium chloride. Prolonged absorption of the injectable compositions can be brought about by the use in the compositions of agents delaying absorption, for example, aluminium monostearate and gelatine.

According to an embodiment, a lyophilized vaccine composition, of the invention is solubilized in water for injection and gently mixed; then an immunoadjuvant, preferably ISA 51, is added; the mixture is gently mixed for emulsification and charged into a suitable syringe. This invention thus also relates to a medical device, including a syringe filled or prefilled with a vaccine composition of the invention. The emulsion is ideally prepared extemporaneously. However, the syringe containing the emulsion can be stored less than 10 hours at 2 - 8 °C. In this case, the emulsion should be allowed to warm up before injecting by friction between the hands.

*[Unit dosage range]*

Another object of the invention is a dosage unit comprising an amount of the immunogenic product ranging from more than 30 mcg to 1000 mcg. In another embodiment, the dosage unit comprises an amount of the immunogenic product ranging from 35 mcg to 1000 mcg. In another embodiment, the dosage unit comprises an amount of the immunogenic product ranging from 35 mcg to 750 mcg. In another embodiment, the dosage unit comprises an amount of the immunogenic product ranging from 35 mcg to 500 mcg. In another embodiment, the dosage unit comprises an amount of the immunogenic product ranging from 35 mcg to 450 mcg. In another embodiment, the dosage unit comprises an amount of the immunogenic product ranging from 35 mcg to 400 mcg. In another embodiment, the dosage unit comprises an amount of the immunogenic product ranging from 35 mcg to 350 mcg. In another embodiment, the dosage unit comprises an amount of the immunogenic product ranging from 35 mcg to 300 mcg. In another embodiment, the dosage unit comprises an amount of the immunogenic product ranging from 35 mcg to 250 mcg. In another embodiment, the dosage unit comprises an amount of the immunogenic product ranging from 35, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390 to 400 mcg.

In another embodiment, the dosage unit comprises an amount of the immunogenic product ranging from 60 mcg to 240 mcg. In another embodiment, the dosage unit comprises an amount of the immunogenic product ranging from 60 mcg to 120 mcg.

In another embodiment, the dosage unit comprises an amount of the immunogenic product of 60 mcg. In another embodiment, the dosage unit comprises an amount of the immunogenic product of 120 mcg. In another embodiment, the dosage unit comprises an amount of the immunogenic product of 240 mcg.

*[Kit and Medical device]*

This invention also pertains to a kit comprising:

- 1 vial (Vial Number 1) containing the immunogenic product of the invention, typically of 3mL;
- 1 vial (Vial Number 2) containing adjuvant, preferably ISA51; this vial is capable of containing 3 mL of adjuvant and may be a container of 8 mL;
- 1 syringe, typically a Braun Injekt-F® of 1 mL;
- 1 needle (Needle Number 1) for emulsion preparation; this needle is preferably a 20G needle;
- 1 needle (Needle Number 2) for injection, preferably intramuscular injection; this needle is preferably a 23G needle.

This invention also pertains to a method for preparing a vaccine from the kit, comprising:

(1) pulling up 0.4 ml of adjuvant from Vial Number 2. Discharge this syringe content into Vial Number 1 containing 0.4 ml of immunogenic product.

(4) pumping up and down the total vial content a sufficient number of times for emulsifying the content, typically 30 times and finally pulling up the whole emulsion.

Prior to injection, Needle Number 1 is preferably switched for Needle Number 2 and air is purged from the syringe.
In one embodiment, said kit comprises:

- 1 vial (Vial Number 1) containing 0.4 ml of the immunogenic product of the invention;
- 1 vial (Vial Number 2) containing at least 0.4 ml of adjuvant, preferably ISA51;
- 1 syringe, typically a Braun Injekt-F® of 1 mL;

- 1 needle (Needle Number 1) for emulsion preparation; this needle is preferably a 20G needle;
- 1 needle (Needle Number 2) for injection, this needle is preferably a 23G needle.

In another embodiment, the immunogenic product is in a lyophilized form. Therefore, the kit comprises:

- 1 vial (Vial Number 1) containing lyophilized product of the invention, typically of 3mL;
- 1 vial (Vial Number 2) containing water for injection typically of 2mL;
- 1 vial (Vial Number 3) containing adjuvant, preferably ISA51; this vial is capable of containing 3 mL of adjuvant and may be a container of 8 mL;
- 1 syringe, typically a Braun Injekt-F® of 1 mL;
- 1 needle (Needle Number 1) for emulsion preparation; this needle is preferably a 20G needle;
- 1 needle (Needle Number 2) for injection, preferably intramuscular injection; this needle is preferably a 23G needle.

This invention also pertains to a method for preparing a vaccine from the kit, comprising:

(1) injecting of water for injection from Vial Number 2 into the Vial Number 1 by using the syringe connected to Needle number 1;
(2) rotating gently Vial Number 1 during 1-5 minutes until complete solubilization of the preparation ;
(3) with the same syringe and needle, pulling up adjuvant from Vial Number 3. Discharge this syringe content into Vial Number 1.
(4) pumping up and down the total vial content a sufficient number of times for emulsifying the content, typically 30 times and finally pulling up the whole emulsion.

This invention also relates to the medical device which is the syringe filled or prefilled with the composition, emulsion or vaccine of the invention.
The invention also relates to a medical device comprising a vial or a carpule prefilled with the product of the invention or with the vaccine composition of the invention.
In one embodiment, the medical device comprises an amount of the immunogenic product ranging from more than 30 mcg to 1000 mcg. In another embodiment, the medical device comprises an amount of the immunogenic product ranging from 35 mcg to 1000 mcg. In another embodiment, the medical device comprises an amount of the immunogenic product ranging from 35 mcg to 750 mcg. In another embodiment, the medical device comprises an amount of the immunogenic product ranging from 35 mcg to 500 mcg. In another embodiment, the medical device comprises an amount of the immunogenic product ranging from 35 mcg to 450 mcg. In another embodiment, the medical device comprises an amount of the immunogenic product ranging from 35 mcg to 400 mcg. In another embodiment, the medical device comprises an amount of the immunogenic product ranging from 35 mcg to 350 mcg. In another embodiment, the medical device comprises an amount of the immunogenic product ranging from 35 mcg to 360 mcg. In another embodiment, the medical device comprises an amount of the immunogenic product ranging from 35 mcg to 250 mcg. In another embodiment, the medical device comprises an amount of the immunogenic product ranging from 35, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390 to 400 mcg.
In another embodiment, the medical device comprises an amount of the immunogenic product ranging from 60 mcg to 240 mcg. In another embodiment, the medical device comprises an amount of the immunogenic product ranging from 60 mcg to 120 mcg.
In another embodiment, the medical device comprises an amount of the immunogenic product of 60 mcg. In another embodiment, the medical device comprises an amount of the immunogenic product of 120 mcg. In another embodiment, the medical device comprises an amount of the immunogenic product of 240 mcg.

**BRIEF DESCRIPTION OF THE DRAWINGS**

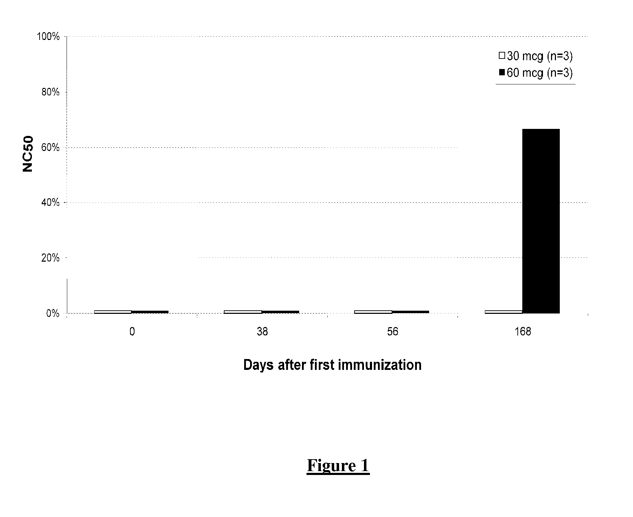

**Figure 1:** Percentage of immunized patient serum samples showing IFNα neutralizing activity.

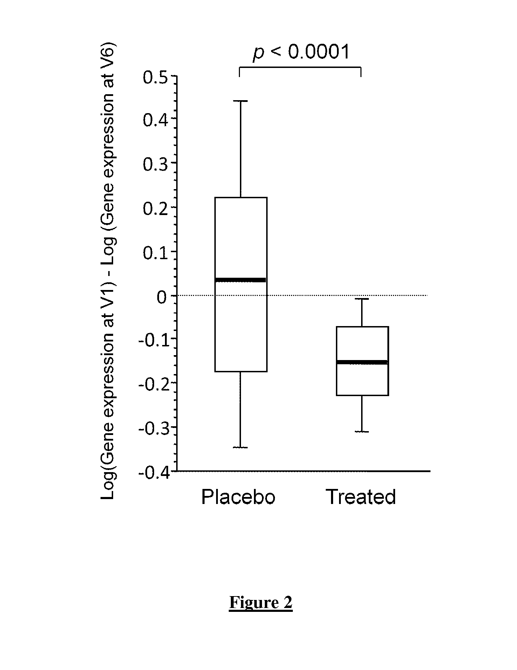

**Figure 2:** Differential evolution of IFN-induced genes in treated- versus placebo patients. Out of 11 patients displaying increased levels of IFN-induced gene expression at baseline, 8 were treated with the immunogenic product and 3 received placebo injections. The levels of 250 IFN-induced genes showing the highest levels of over-expression in SLE patients were evaluated using high-density microarrays. The results are depicted as the mean log2(level of expression at V1) - log2(level of expression at V6). The *p* value was calculated using a Student's t-test.

**EXAMPLES**

**EXAMPLE 1: Preparation of the immunogenic product**

Keyhole Limpet Hemocyanin (KLH) was extracted from the lymph of the marine gastropod mollusk *Megathura crenulata* and then purified under GMP condition. Results from stability assays performed in storage conditions at a temperature of 2-8°C showed that the shelf life of the purified KLH is of 36 months at 2-8°C.
Recombinant human IFNα 2b was produced in *E. coli* under GMP conditions.
Batches of the product of the invention at 350 mg IFNα scale were produced using the manufacturing process developed below.

**a) Conjugation with Glutaraldehyde**

The filtered KLH is added to the IFNα 2b solution with a IFNα:KLH ratio of 20:1, (corresponding to a molar ratio of 20 monomer of IFNα for 1 subunit of KLH) based on UV concentration.
The conjugation is carried out with glutaraldehyde (added to reach 22.5 mM in the reaction medium) and the solution is mixed during 45 min at $23 \pm 2$ °C.

**b) Quenching with glycine**

The reaction is quenched with Glycine 0.1 M during 30 min.

**c) First tangential flow filtration (TFF 1)**

The first TFF is performed with a Pall Minim II TFF system and a polyethersulfone membrane of 0.02 $m^2$ with a molecular weight cut off of 10 kDa sanitized with 0.5 M NaOH and equilibrated with the working buffer.
The quenched solution is then clarified by 0.22 pm-filtration. The intermediate is diluted twice in working buffer and then diafiltered by tangential flow filtration (TFF) and 12 volumes of working buffer. The retentate is harvested and is stored for less than 20 hours.

**d) Inactivation with Formaldehyde**

Formaldehyde is added to the retentate to reach a final concentration of 66.6 mM using a peristaltic pump. The inactivation reaction is performed during 40 hours in an incubator set to $37\pm2$°C with a daily mixing of the solution with a magnetic stirrer.

**e) Quenching with glycine**

The reaction is then quenched with 0.1 M of Glycine during 30 min.

**f) Second tangential filtration (TFF 2)**

The second TFF is performed with a Pall Minim II TFF system and a polyethersulfone membrane of 0.02 $m^2$ with a molecular weight cut off of 100 kDa sanitized with 0.5 M NaOH and equilibrated with the formulation buffer.
The quenched solution is clarified by 0.2 $\mu$m filtration. The intermediate is concentrated to have a starting tangential volume of $\approx$ 900 mL and next filtrated by TFF with 12 volumes of formulation buffer (1.47 mM KH2PO4, 8.1 mM Na2HPO4, 2.68 mM KCl, 136.9 mM NaCl, pH 7.3) to eliminate the low molecular weight homopolymers of IFNα and the non reactive reagents. The retentate is harvested and then diluted to a theoretical concentration of 300 $\mu$g/mL based on concentration determination by Bradford protein assay and then 0.2$\mu$m-filtered to obtain the immunogenic product of the invention.

**EXAMPLE 2: antigenicity of the product**

A sandwich ELISA anti IFNα/KLH was carried out as following. Briefly, a 96 wells plate was coated with the capture antibody: rabbit polyclonal anti-KLH antibody, and blocked with a blocking buffer (such as casein 2% in PBS for example) during 90 min at 37°C. The plate was incubated during 90 min at 37°C the plate with a dilution series of the immunogenic product from 250 ng/ml to 8 two fold dilutions or with negative controls such as KLH and IFNα. A detection antibody such as for example a biotinylated anti-IFNα antibody was then added for 90 min. Finally the plate was incubated with streptavidin-HRP during 30 min at 37°C and the complex developed with an o-phenylenediamine dihydrochloride (OPD) substrate solution during 30 min. After stopping the enzymatic reaction, the intensity of the resulting color is determined by spectrophotometric methods at 490 nm.

This test confirmed that the product comprises IFN$\alpha$ that is antigenic, i.e. recognized by anti- IFN$\alpha$ antibody and that said IFN$\alpha$ is coupled to KLH.

## EXAMPLE 3: Immunogenicity of the product (TEST A)

4 $\mu$g of total proteins of the product as determined by Bradford protein assay were injected to 7 Balb/c mice of 6-8 weeks at day 0 and day 21.

At day 31, mice were bleeded and the sera were harvested.

An anti-IFN$\alpha$ ELISA was carried out on preimmune and harvested sera as following:

- a 96 wells plate was coated with 100 ng of IFN$\alpha$ -2b and incubated overnight at 2°C-8°C,
- a blocking buffer was added during 90 min at 37°C,
- the immunogenic product was added at a dilution of 1/2500 up to at least 8 two fold dilutions and the plate was incubated during 90 min at 37°C,
- the plate was incubated with an anti-mouse immunoglobulin labeled antibody such as an HRP conjugated antibody during 90 min at 37°C,
- the ELISA was developed with an o-phenylenediamine dihydrochloride (OPD) substrate solution. After stopping the enzymatic reaction, the intensity of the resulting color is determined by spectrophotometric methods at 490 nm.

This test demonstrated that in the 7 mice, immunization with the immunogenic product led to the presence of anti-IFN$\alpha$ antibodies titers.

## EXAMPLE 4: Residual activity of the product (TEST B)

This assay was based on the protective effect of IFN$\alpha$ on the cytopathic effect (CPE) of Vesicular Stomatitis Virus (VSV) on Madin-Darby Bovine Kidney (MDBK) cells.

The immunogenic product and the recombinant IFN$\alpha$ 2b used for preparing the immunogenic product (positive control) were diluted at at least 500 ng/ml and at least 1000 U/ml respectively in Basal medium (RPMI supplemented with 2 mM glutamine, 1 mM sodium pyruvate, 1 mM Hepes). 50 $\mu$l of the immunogenic product and the positive control were plated in a 96 wells plate and diluted in a series of two fold dilutions in the Basal medium. 2 10$^4$ MDBK cells were added in each well in 50 $\mu$l of Cell medium (RPMI supplemented with 4% FBS, 2 mM glutamine, 1 mM sodium pyruvate and 1 mM Hepes) and the plate was incubated overnight at 37°C, 5% $CO_2$. The virus was then diluted in Basal medium to at least 20000 TCID$_{50}$ (Tissue Culture Infection Dose 50: 10 times the dilution to kill 50% of infected cells). The plate was emptied and 100 $\mu$l of the diluted virus was added. The plate was then incubated overnight at 37°C, 5% $CO_2$.

At the end of the culture, 20 $\mu$l/well of a solution of MTS/PMS (100 $\mu$l MTS/5 $\mu$l PMS; Promega G5430) were added to the wells and the plate was incubated for another 4h at 37°C 5% CO2. The plate was then read at 490 nm on a spectrophotometer.

The percentage of antiviral activity of the immunogenic product was calculated and for the two batches of product tested, the antiviral activity was less than 1% of the antiviral activity of IFN$\alpha$.

## EXAMPLE 5: Neutralization capacity of the product (TEST C)

The neutralizing capacity of the product was assessed by evaluating the cell viability in presence of the vesicular stomatitis virus replicating in MDBK cells.

4$\mu$g of total proteins (as determined by a Bradford protein assay) of the immunogenic product were injected in Balb/c mice of 6-8 weeks at day 0 and day 21. A serum sample was obtained before immunization (pre-immune serum sample) and at day 31 (test serum sample).

25 $\mu$l of pre-immune and test serum samples were plated in a 96-well plate at a dilution of 1/200 up to 8 dilutions from 1/200. The positive control (polyclonal anti-IFN$\alpha$ from PBL, Piscataway, NJ, ref.31100-1) was diluted from 3125 UI/well to 100 UI/well in Basal medium (RPMI supplemented with 2 mM glutamine, 1 mM sodium pyruvate and 1 mM hepes) and 25 $\mu$l were also plated in the plate.

25 U/well (final concentration) in 25 $\mu$l of basal medium of IFN$\alpha$ was added to each well and the plate is incubated for 60 min at room temperature.

20000 MDBK cells in Assay medium (RPMI supplemented with 4% FBS, 2 mM glutamine, 1 mM sodium pyruvate, 1 mM hepes) were added to each well and the plate was incubated overnight at 37°C, 5% $CO_2$.

The virus was diluted to at least 20000 $TCID_{50}$ (10 times the dilution to kill 50% of infected cells) in virus medium (RPMI supplemented with 2 mM glutamine, 1 mM sodium pyruvate, 1 mM hepes). The plate was emptied and 100 μl of virus was added to each well before incubation for 24h at 37°C, 5% $CO_2$.

At the end of the culture, 20 μl/well of a solution of MTS/PMS (100 μl MTS/5 μl PMS; Promega G5430) were added to the wells and the plate was incubated for another 4h at 37°C 5% CO2. The plate was then read at 490 nm on a spectrophotometer.

The NC50 was calculated for all the 7 test samples: mean NC50 = 253789 IU/ml (SEM = 172526), demonstrating that all serum comprised antibodies anti-IFNα capable of neutralizing the antiviral activity of IFNα.

## EXAMPLE 6: Examples of compositions and vaccine comprising the immunogenic product

One illustrative composition comprising the immunogenic product is described in Table 1.

**Table 1**

| Components | Quantity |
|---|---|
| Product of the invention | 160 μg |
| di-sodium phosphate | 8.95 mg |
| Disodium dihydrogen phosphate | 805 μg |

One illustrative vaccine comprising the immunogenic product is described in Table 2.

**Table 2**

| Emulsion | |
|---|---|
| Components | Quantity |
| Product of the invention | 160 μg |
| di-sodium phosphate | 8.95 mg |
| Disodium dihydrogen phosphate | 805 μg |
| Drakeol 6VR (mineral oil) | 0.30 g |
| Montanide 80 (mannide monooleate) | 0.04 g |
| Total volume | 0.8 ml |

## EXAMPLE 7: Clinical trial

A clinical trial was carried out using the vaccine composition as described in Table 2.

*Study design:*

3 administrations of the product were performed at day 0, day 7 and day 28 in adults subjected to SLE.
The following doses of the product were tested: 30 mcg, 60 mcg, 120 mcg and 240 mcg.

*Study population:*

28 male or female patients aged between 18 and 50 years, with mild to moderate SLE (SLEDAI 4-10), active disease despite receiving treatment. A normal control interferon gene signature was established in 48 healthy volunteers. PBMC of 18 out of the 48 healthy volunteers were stimulated in vitro with type I interferons in order to identify an interferon signature on the high-density arrays. A SLE signature was established by comparing the signatures between healthy volunteers and SLE patients at baseline.
An interim analysis was performed in the patients enrolled in the first three groups, ie having received the 30, 60 or 120 mcg doses or placebo.

**Table 3: Demographics for enrolled patients**

| Summary Statistics | | 30 mcg (N = 3) | 60 mcg (N = 6) | 120 mcg (N = 6) | Placebo (N = 5) | Total (N = 20) |
|---|---|---|---|---|---|---|
| Age (years) | | | | | | |
| | Mean (SD) | 36.0(9.85) | 39.3 (3.98) | 34.2 (12.12) | 38.6(11.52) | 37.1 (9.28) |
| | Median | 33.0 | 38.0 | 32.5 | 43.0 | 37.5 |
| Sex, n(%) | | | | | | |
| | Male | 0 (0.0) | 0 (0.0) | 0 (0.0) | 0 (0.0) | 0 (0.0) |
| | Female | 3(100.0) | 6(100.0) | 6(100.0) | 5(100.0) | 20(100.0) |
| Race, n(%) | | | | | | |
| | White-Caucasian | 3(100.0) | 6(100.0) | 6(100.0) | 5(100.0) | 20(100.0) |
| SLEDAI-2000 | | | | | | |
| | Mean | 8.67 (1.15) | 7.50(2.81) | 6.00(2.19) | 8.80(1.09) | |
| | Median | 8.00 | 8.50 | 6.00 | 8.00 | |
| Anti-dsDMAab | | | | | | |
| | Mean (SD) | 53.93(58.22) | 61.25 (113.46) | 140.55 (242.63) | 88.70 (113.62) | |
| | Median | 33.10 | 15.45 | 23.60 | 40.90 | |
| DURATION OF DISEASE (YEARS) | | | | | | |
| | Mean (SD) | 10.0(2.18) | 8.9 (8.82) | 7.3(5.99) | 5.9 (4.75) | 7.9 (6.11) |
| | Median | 11.0 | 6.1 | 6.1 | 3.6 | 6.4 |
| CONCOMMITANT CORTICOSTEROIDS | | 100% | 66.7% | 83.3% | 100% | |

*Results*

Interim results on patients administrated with 30, 60, and 120 mcg of the immunogenic product are presented.

*Safety and tolerability of the vaccine*

No product-related SAEs have been reported in this analysis and no patient withdrew from the trial for tolerability or safety reasons. Regular interim safety analyses were performed by an independent safety board. No clinically significant change in laboratory parameters has been detected (hematology, biochemistry, urine).

*Immunogenicity of the vaccine*

Anti-IFNα antibody titers were measured by ELISA from serum samples obtained from the patients.

An anti-IFNα ELISA was carried out as described here above.

Results show that anti-IFNα antibody titers were detected in 80% of the patients treated with the immunogenic product.

*Neutralization activity of the vaccine*

The neutralization activity was assessed in vitro using the following method:

50 μl of serum samples obtained from the patients sera were plated in a 96-well plate at a dilution of 1/200 up to 8 dilutions from 1/200.

The positive control (polyclonal anti-IFN from PBL Piscataway, NJ, 31100-1) was diluted from 100 ng/well to 3.125 ng/well and 50μl were added to the plate. Dilutions were carried out in Basal medium (RPMI supplemented with 2 mM glutamine, 1 mM sodium pyruvate and 1 mM hepes).

10 U/well (final concentration) of IFNα 2b were added to each well and the plate was incubated for 60 min at room temperature.

30000 MDBK cells in Assay medium (RPMI supplemented with 4% FBS, 2 mM glutamine, 1 mM sodium pyruvate, 1 mM hepes) were added to each well and the plate was incubated overnight at 37°C, 5% $CO_2$.

The virus was diluted to at least 63 $TCID_{50}$ (10 times the dilution to kill 50% of infected cells) in virus medium (RPMI supplemented with 2 mM glutamine, 1 mM sodium pyruvate, 1 mM hepes). The plate was emptied and 100 $\mu$l of virus was added to each well before incubation for 24h at 37°C, 5% $CO_2$.

At the end of the culture, 20 $\mu$l/well of a solution of MTS/PMS (100 $\mu$l MTS/5 $\mu$l PMS; Promega G5430) were added to the wells and the plate was incubated for another 4h at 37°C 5% CO2. The plate was then read at 490 nm on a spectrophotometer.

The results showed that none of the sera from patients treated with 30 mcg of the immunogenic product presents anti-IFN$\alpha$ antibodies having a neutralizing capacity at day 168 after immunization, whereas the sera from patients treated with 60 mcg of the immunogenic product present anti-IFN$\alpha$ antibodies having a neutralizing capacity at day 168 (Figure 1).

These results demonstrated that treatment with more than 30 mcg of the immunogenic product is necessary for having an in vivo neutralization of IFN$\alpha$.

**EXAMPLE 8: Pharmacogenomic analysis**

PBMC were harvested at several time-points before and after injection of the immunogenic product. For this interim analysis, total RNA was extracted at V1 (day 0) and V6 (day 38 after the first injection) samples, labeled according to standard Affymetrix protocol, and hybridized on Genechip HGU133 Plus 2.0 arrays. Statistical analyses were performed on GeneSpring after RMA (Robust Microarray Analysis) normalization of the samples.

Unsupervized clustering algorithms were performed on the baseline samples, and grouped the patients in two categories : those with (n=11), and those without (n=7) the presence of an 'Interferon- signature' at baseline, i.e. the spontaneous over-expression of genes induced by type I interferon (the IFN-induced genes were identified experimentally, based on microarray analyses of IFN-stimulated control PBMC). Not surprisingly, dsDNA titers were significantly higher in the patients with the signature (mean +/- SEM: 131.1. +/- 50.1 UI/ml), compared to the patients without (mean +/-SEM: 44.7 +/- 33.3, p = 0.006 by Mann-Whitney test). Measurable anti-IFN$\alpha$ antibodies were found in 8 follow-up samples of the 8 patients with an IFN signature at baseline who received the immunogenic product, while this was only the case in 2 out of 6 patients without IFN signature treated with the immunogenic product, and none out of the 4 placebo-treated individuals (p = 0.002 by Chi-squared test). Out of the 11 patients with a baseline IFN-signature, 2 received the 30 mcg dose, 1 received the 60 mcg dose, 5 received the 120 mcg dose and 3 were treated with a placebo injection. The changes observed in the expression of the IFN-induced genes between V1 and V6 were significantly different in the patients treated with the immunogenic product, as compared to the patients treated with the placebo (Figure 2).

This result suggests that the immunogenic product has an effect on the expression of IFN-induced genes in vivo.

**Claims**

1. An immunogenic product comprising IFN$\alpha$ coupled to a carrier protein molecule for use in preventing or treating an IFN$\alpha$ related condition in a subject in need thereof, wherein the therapeutically effective amount of the immunogenic product to be administrated to the subject is more than 30 mcg of immunogenic product per administration.

2. The immunogenic product according to claim **1,** wherein the administration of the therapeutically effective amount of the immunogenic product prevents the occurrence of symptoms of a disease linked to an over-production of IFN$\alpha$.

3. The immunogenic product according to claim **1**, wherein the administration of the therapeutically effective amount of the immunogenic product prevents the flare of a disease linked to an over-production of IFN$\alpha$.

4. The immunogenic product according to anyone of claims **1** to **3**, wherein the IFN$\alpha$ related conditions comprise systemic lupus erythematosus, rheumatoid arthritis, scleroderma, Sjögren syndrome, vasculitis, HIV, type I diabetes, autoimmune thyroiditis and myositis.

5. The immunogenic product according to anyone of claims **1** to **4**, wherein the therapeutically effective amount of the immunogenic product to be administrated to the subject is from 35 mcg to 1000 mcg of immunogenic product per administration.

6. The immunogenic product according to anyone of claims **1** to **5**, wherein the immunogenic product is administrated to the subject at least twice in a month.

7. The immunogenic product according to anyone of claims **1** to **6**, wherein the immunogenic product is further administrated to the subject at least once every three months.

8. The immunogenic product according to anyone of claims **1** to **6**, wherein the immunogenic product is further administrated to the subject when, in a serum sample obtained from the subject, the amount of anti- IFNα antibodies is undetectable.

9. The immunogenic product according to anyone of claims **1** to **8**, wherein the immunogenic product is strongly inactivated, which means that the product shows less than 5% of antiviral activity in the conditions of TEST B.

10. The immunogenic product according to anyone of claims **1** to **9**, wherein the immunogenic product is capable of neutralizing the antiviral activity of IFNα in the conditions of TEST C.

11. The immunogenic product according to anyone of claims **1** to **10**, comprising at least one subtype of IFNα.

12. The immunogenic product according to anyone of claims **1** to **11**, wherein the subtype of IFNα is IFNα 2b and the carrier protein molecule is KLH.

13. The immunogenic product according to anyone of claims **1** to **11**, wherein the immunogenic product is a vaccine, preferably in the form of an emulsion.

14. A unit dosage form comprising more than 30 mcg of an immunogenic product comprising IFNα coupled to a carrier protein molecule according to any one of claims **1** to **13**.

15. A medical device comprising more than 30 mcg of an immunogenic product comprising IFNα coupled to a carrier protein molecule according to any one of claims **1** to **13**.

16. A kit comprising at least one vial containing more than 30 mcg of an immunogenic product comprising IFNα coupled to a carrier protein molecule according to any one of claims **1** to **13**, at least one vial containing adjuvant, and means for contacting said solution to the adjuvant, and for emulsifying the mixture of the aqueous solution with the adjuvant.

**Figure 1**

**Figure 2**

| | Europäisches Patentamt European Patent Office Office européen des brevets | EUROPEAN SEARCH REPORT | Application Number EP 11 30 5408 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | MATHIAN ALEXIS ET AL: "Active immunisation of human interferon alpha transgenic mice with a human interferon alpha Kinoid induces antibodies that neutralise interferon alpha in sera from patients with systemic lupus erythematosus", ANNALS OF THE RHEUMATIC DISEASES, vol. 70, no. 6, June 2011 (2011-06), pages 1138-1143, XP009152971, * Abstract, Introduction, Methods (IFN-K and polymerised IFN[alpha], Injection of mide with saline or IFNgf or IFN-K), Results (Antibodies induced by IFN-K immunisation neutralise huIFN[alpha]2b, Antibodies induced by IFN-K neutralise IFN[alpha] activity in sea from patients with active SLE), Discussion * ----- | 1-16 | INV. A61K38/21 A61K39/00 |
| X | US 2007/202102 A1 (BIZZINI BERNARD [FR] ET AL) 30 August 2007 (2007-08-30) * paragraphs [0008], [0015], [0032], [0035] * * paragraphs [0042] - [0052] * ----- -/-- | 1-16 | TECHNICAL FIELDS SEARCHED (IPC) A61K |

The present search report has been drawn up for all claims

| Place of search | Date of completion of the search | Examiner |
|---|---|---|
| Munich | 26 October 2011 | Page, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt
European
Patent Office
Office européen
des brevets

## EUROPEAN SEARCH REPORT

**Application Number**

EP 11 30 5408

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CROW, MARY K.: "Interferon-alpha: A Therapeutic Target in Systemic Lupus Erythematosus", PubMed Central (PMC) Author Manuscript RHEUM DIS CLIN NORTH AM, vol. 36, no. 1 1 February 2010 (2010-02-01), pages 1-13, XP002661031, Retrieved from the Internet: URL:http://www.ncbi.nlm.nih.gov/pmc/articl es/PMC2843146/pdf/nihms175764.pdf [retrieved on 2011-10-26] * page 3, paragraph 2 * * page 7, paragraph 2.3 * * page 9, paragraph 3 * | 1-16 | |
| A | YAO Y ET AL: "Neutralization of interferon-[alpha]/[beta]-inducible genes and downstream effect in a phase I trial of an anti-interferon-[alpha] monoclonal antibody in systemic lupus erythematosus", ARTHRITIS & RHEUMATISM, vol. 60, no. 6, 1 June 2009 (2009-06-01), pages 1785-1796, XP009147413, JOHN WILEY & SONS, INC, US ISSN: 0004-3591, DOI: 10.1002/ART.24557 [retrieved on 2009-05-28] * Introduction, Results (Anti-IFN-[alpha] mAb neutralizes overexpression of IFN-[alpha]/[beta]-inducible genes in whole blood from SLE patients in a dose-dependent manner), Discussion * | 1-16 | TECHNICAL FIELDS SEARCHED (IPC) |

-/--

The present search report has been drawn up for all claims

| Place of search | Date of completion of the search | Examiner |
|---|---|---|
| Munich | 26 October 2011 | Page, Michael |

Europäisches
Patentamt

European
Patent Office

Office européen
des brevets

# EUROPEAN SEARCH REPORT

**Application Number**

EP 11 30 5408

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARY K CROW: "Type I interferon in organ-targeted autoimmune and inflammatory diseases", ARTHRITIS RESEARCH & THERAPY, vol. 12, no. Suppl 1, 1 January 2010 (2010-01-01), pages S5-S5, XP055009181, ISSN: 1478-6354, DOI: 10.1186/ar2886 * the whole document *<br><br>----- | 1-16 | |
| | | | **TECHNICAL FIELDS SEARCHED     (IPC)** |

The present search report has been drawn up for all claims

| Place of search | Date of completion of the search | Examiner |
|---|---|---|
| Munich | 26 October 2011 | Page, Michael |

EPO FORM 1503 03.82 (P04C01)

**ANNEX TO THE EUROPEAN SEARCH REPORT
ON EUROPEAN PATENT APPLICATION NO.**

EP 11 30 5408

This annex lists the patent family members relating to the patent documents cited in  the above-mentioned European search report.
The members are as contained in the European Patent Office EDP file on
The European Patent Office is in no way liable for these particulars which are merely given for the purpose of information.

26-10-2011

| Patent document cited in search report | Publication date | Patent family member(s) | | Publication date |
|---|---|---|---|---|
| US 2007202102 A1 | 30-08-2007 | AT | 495756 T | 15-02-2011 |
| | | CA | 2539172 A1 | 31-03-2005 |
| | | CN | 1882610 A | 20-12-2006 |
| | | DK | 1668036 T3 | 09-05-2011 |
| | | EP | 1668036 A2 | 14-06-2006 |
| | | EP | 2105448 A2 | 30-09-2009 |
| | | ES | 2358118 T3 | 05-05-2011 |
| | | FR | 2859725 A1 | 18-03-2005 |
| | | WO | 2005028513 A2 | 31-03-2005 |
| | | JP | 2007533622 A | 22-11-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 109942 A
- EP 180564 A
- EP 231039 A
- GB 2189141 A
- US 4877612 A

**Non-patent literature cited in the description**

- **VAN BOXEL-DEZAIRE et al.** *Immunity,* 2006, vol. 25, 361-372
- **BACCALA et al.** *Immunol Rev,* 2005, vol. 204, 9-26
- **DALL'ERA et al.** *Ann Rheum Dis,* 2005, vol. 64, 1692-7
- **SEDAGHAT et al.** *J. Virol.,* 2008, vol. 82 (4), 1870-1883
- **MANDL et al.** *Nat. Med.,* 2008
- **DE GROOT et al.** *Trends. Immunol.,* 2007, vol. 28 (11
- **PESTKA et al.** *Immunological reviews,* 2004, vol. 202, 8-32
- **MIRE-SLUIS et al.** *J. Immunol Meth.,* 2004, vol. 289, 1-16
- **MCGHEE, J. R. et al.** On vaccine development. *Sem. Hematol.,* 1993, vol. 30, 3-15
- **BOVARNIK et al.** *J. Bacteriology,* 1950, vol. 59, 509